# EUROPEAN PATENT APPLICATION

(11) **EP 4 721 744 A1**
(43) Date of publication of application: **08.04.2026**
(21) Application number: 24814577.3
(22) Date of filing: 31.05.2024
(51) Int. Cl.: A61K 31/546, A61K 47/18, A61K 9/19, A61P 31/04

(54) **PHARMACEUTICAL COMPOSITION COMPRISING CEPHALOSPORIN ANTIBACTERIAL COMPOUND**

(30) Priority: 01.06.2023 CN 202310642200
(71) Applicant: Jiangsu Hengrui Pharmaceuticals Co., Ltd., Lianyungang, Jiangsu 222047 (CN)
(72) Inventor: FAN, Yingfang, Lianyungang, Jiangsu 222047 (CN); JIANG, Kai, Lianyungang, Jiangsu 222047 (CN); YU, Xinxin, Lianyungang, Jiangsu 222047 (CN); YE, Linmao, Lianyungang, Jiangsu 222047 (CN); AN, Dong, Lianyungang, Jiangsu 222047 (CN); CHEN, Hao, Lianyungang, Jiangsu 222047 (CN)
(74) Representative: Regimbeau
(86) International application number: PCT/CN2024/096563
(87) International publication number: WO 2024/245383

(57) **Abstract**

A pharmaceutical composition comprising a cephalosporin antibacterial compound, which pharmaceutical composition comprises a compound as shown in formula (I), or a pharmaceutically acceptable salt thereof, and arginine. The composition has good stability and can be better used in clinical practice.

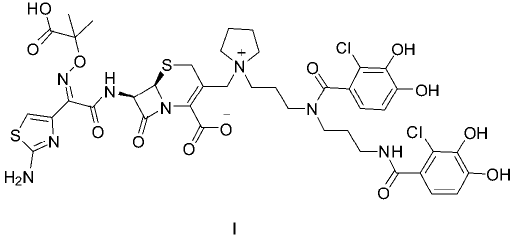

## Description

### Technical Field

The present disclosure belongs to the field of pharmaceutical formulations, and in particular relates to a pharmaceutical composition comprising a cephalosporin antibacterial compound and a preparation method therefor.

### Background Art

The development of antibacterial treatment regimens has become an ongoing challenge faced by today's society. Antibacterial drugs include various antibiotics, sulphonamides, imidazoles, nitroimidazoles, quinolones and other chemically synthetic drugs. Among them, β-lactam antibiotics represent a very important category. Up to now, a variety of β-lactamase drugs have been reported in literature studies or launched on the market, and they have become extremely important antibacterial drugs in clinical practice. However, the problem of resistance to β-lactam drugs is also becoming increasingly serious, and a growing number of bacteria have developed drug resistance by producing β-lactamase to degrade β-lactamase drugs.

The emergence of Gram-negative bacteria that have become highly resistant to β-lactamase drugs (including cephalosporins and carbapenems) has posed significant clinical application challenges. Such drug resistance is caused by the production of class A or D serine β-lactamases (which have extended substrate spectrums) and class B metallo-β-lactamases. Metallo-β-lactamases are known to be one of the causes of multidrug resistance in Gram-negative bacteria. However, the development of cephalosporin compounds with more potent antibacterial activity, especially those effective against Gram-negative bacteria producing multiple β-lactamases, remains a challenge in the current field of antibacterial drug research.

According to literature reports, cephalosporin compounds containing catechol groups in their molecules exhibit potent antibacterial activity against Gram-negative bacteria. The mechanism of action is as follows: the catechol groups in the molecules form chelates with extracellular Fe³⁺, which enables the compound to be efficiently transported into bacterial cells via the Fe³⁺ transport system (tonB-dependent transport system) on the cell membrane. This Trojan horse strategy increases the concentration of the compound in the periplasmic space (the narrow space between the outer membrane and the cell wall), enabling the compound to bind to a receptor and thereby inhibit the synthesis of the bacterial cell wall. Therefore, research has been conducted on compounds that have a catechol or similar structure in the 3-position side chain or 7-position side chain of the cephalosporin scaffold (EP 0416410 B1). Cefiderocol is a novel siderophore cephalosporin (WO 2010050468, WO 2017216765, etc.). The FDA (U.S. Food and Drug Administration) has approved Fetroja (cefiderocol) developed by Shionogi & Co., Ltd. for the treatment of complicated urinary tract infections (cUTI) in patients aged 18 years and older, including kidney infections caused by susceptible Gram-negative bacteria.

WO 2022152146 discloses a siderophore cephalosporin having a structure as shown in formula (I) and showing good antibacterial activity. However, the compound has extremely poor stability, so the bulk compound needs to be stored at or below -60°C, which poses a challenge to the manufacture of formulation products.

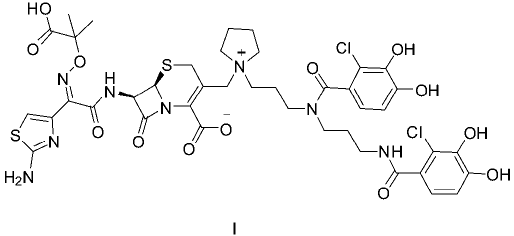

### Summary of the Invention

The object of the present disclosure is to provide a pharmaceutical composition comprising a cephalosporin antibacterial compound, which composition has excellent stability.

In one aspect, the present disclosure provides a pharmaceutical composition comprising a compound as shown in formula (I) or a pharmaceutically acceptable salt thereof, and arginine,

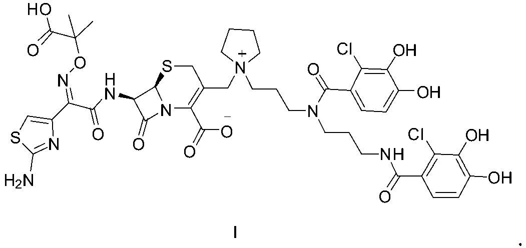

In some embodiments, the concentration of the compound as shown in formula (I) or the pharmaceutically acceptable salt thereof is 1 mg/mL to 200 mg/mL, and non-limiting examples include 1 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, 200 mg/mL and any range between these point values. In some embodiments, the concentration of the compound as shown in formula (I) or the pharmaceutically acceptable salt thereof is 10 mg/mL to 180 mg/mL, or 30 mg/mL to 180 mg/mL, or 30 mg/mL to 150 mg/mL.

With respect to the concentration of the compound as shown in formula (I) or the pharmaceutically acceptable salt thereof of the present disclosure, if the active substance is a pharmaceutically acceptable salt of the compound as shown in formula (I), the concentration of the pharmaceutically acceptable salt of the compound as shown in formula (I) is calculated based on the concentration of the compound as shown in formula (I) in a free form.

In some embodiments, the weight-to-volume ratio of the arginine is 0.01% to 30%, or 0.1% to 20%, or 1% to 15%, and non-limiting examples include 0.02%, 0.05%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.2%, 2.4%, 2.6%, 2.8%, 3.0%, 4.0%, 5.0%, 6.0%, 7.0%, 8.0%, 9.0%, 10.0%, 11.0%, 12.0%, 13.0%, 14.0%, 15.0%, 18.0%, 20.0%, 22.0%, 25.0%, 28.0%, 30.0% and any range between these point values.

In some embodiments, the concentration of the arginine is 1 mg/mL to 300 mg/mL, and non-limiting examples include 1 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, 200 mg/mL, 210 mg/mL, 220 mg/mL, 230 mg/mL, 240 mg/mL, 250 mg/mL, 260 mg/mL, 270 mg/mL, 280 mg/mL, 290 mg/mL, 300 mg/mL and any range between these point values; In some embodiments, the concentration of the compound as shown in formula (I) or the pharmaceutically acceptable salt thereof is 10 mg/mL to 250 mg/mL, or 30 mg/mL to 200 mg/mL, or 30 mg/mL to 180 mg/mL.

In some embodiments, the weight ratio of the compound as shown in formula (I) or the pharmaceutically acceptable salt thereof to the arginine may be 1 : 0.1-1 : 20, e.g., 1 : 0.1, 1 : 0.2, 1 : 0.3, 1 : 0.4, 1 : 0.5, 1 : 0.6, 1 : 0.7, 1 : 0.8, 1 : 0.9, 1 : 1, 1 : 1.1, 1 : 1.2, 1 : 1.3, 1 : 1.4, 1 : 1.5, 1 : 1.6, 1 : 1.7, 1 : 1.8, 1 : 1.9, 1 : 2, 1 : 3, 1 : 4, 1 : 5, 1 : 6, 1 : 7, 1 : 8, 1 : 9, 1 : 10, 1 : 12, 1 : 14, 1 : 16, 1 : 18, 1 : 20 and any range between these point values.

The arginine in the pharmaceutical composition of the present disclosure may comprise a free form of arginine, a salt form of arginine, and a mixed form of the free form and salt form of arginine. Unless otherwise specified, the content or proportion of the arginine of the present disclosure generally refers to the total content or proportion of all forms of arginine, calculated based on free arginine.

In some embodiments, the arginine is free arginine and/or arginine hydrochloride. In some embodiments, the weight ratio of the free arginine to the arginine hydrochloride may be 1 : 0.1 to 1 : 20, e.g., 1 : 0.1, 1 : 0.2, 1 : 0.3, 1 : 0.4, 1 : 0.5, 1 : 0.6, 1 : 0.7, 1 : 0.8, 1 : 0.9, 1 : 1, 1 : 2, 1 : 3, 1 : 4, 1 : 5, 1 : 6, 1 : 7, 1 : 8, 1 : 9, 1 : 10, 1 : 12, 1 : 14, 1 : 16, 1 : 18, 1 : 20 and any range between these point values.

In some embodiments, the pharmaceutical composition may further comprise other protective excipients, wherein the protective excipients are selected from one or more of amino acids, saccharides, high-molecular-weight polysaccharides, cyclodextrins and inorganic salts, including but not limited to, one or more of histidine, lysine, glycine, sucrose, glucose, lactose, trehalose, maltose, inositol, hydroxyethyl starch, PEG, dextran, sulfobutyl-β-cyclodextrin and sodium chloride.

In some embodiments, the weight-to-volume ratio of the protective excipient is 0.01% to 30%, or 0.1% to 20%, or 1% to 15%.

In some embodiments, the pharmaceutical composition further comprises sodium chloride. The concentration of the sodium chloride may be 1 mg/mL to 100 mg/mL, and non-limiting examples include 1 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 31 mg/mL, 32 mg/mL, 33 mg/mL, 34 mg/mL, 35 mg/mL, 36 mg/mL, 37 mg/mL, 38 mg/mL, 39 mg/mL, 40 mg/mL, 50 mg/mL, 60 mg/mL, 70 mg/mL, 80 mg/mL, 90 mg/mL, 100 mg/mL and any range between these point values. In some embodiments, the concentration of the sodium chloride is 1 mg/mL to 80 mg/mL, or 5 mg/mL to 60 mg/mL, or 5 mg/mL to 50 mg/mL.

In some embodiments, the weight ratio of the compound as shown in formula (I) or the pharmaceutically acceptable salt thereof to the sodium chloride may be 1 : 0.01-1 : 10, e.g., 1 : 0.01, 1 : 0.02, 1 : 0.03, 1 : 0.04, 1 : 0.05, 1 : 0.06, 1 : 0.07, 1 : 0.08, 1 : 0.09, 1 : 0.1, 1 : 0.2, 1 : 0.3, 1 : 0.4, 1 : 0.5, 1 : 0.6, 1 : 0.7, 1 : 0.8, 1 : 0.9, 1 : 1, 1 : 1. 1, 1 1. 2, 1 : 1.3, 1 : 1.4, 1 1.5, 1 : 1.6, 1 : 1.7, 1 : 1.8, 1 : 1.9, 1 : 2, 1 : 3, 1 : 4, 1 : 5, 1 : 6, 1 : 7, 1 : 8, 1 : 9, 1 : 10 and any range between these point values.

In some embodiments, the pharmaceutical composition further comprises a buffer. The buffer is selected from an acetate buffer, a citrate buffer, a histidine buffer and a succinate buffer. In some embodiments, the buffer is selected from acetic acid-sodium acetate, histidine-acetic acid, histidine-histidine hydrochloride, citric acid-sodium citrate, succinic acid-histidine and succinic acid-sodium succinate buffers.

In some embodiments, the concentration of the buffer in the pharmaceutical composition is 1 mM to 50 mM, and non-limiting examples include 1 mM, 3 mM, 5 mM, 10 mM, 12 mM, 15 mM, 16 mM, 17 mM, 18 mM, 19 mM, 20 mM, 30 mM, 40 mM, 50 mM and any range between these point values.

In some embodiments, the pharmaceutical composition may further comprise a pH regulator. The pH regulator includes, but is not limited to, sodium hydroxide, hydrochloric acid, etc.

In some embodiments, the pH of the pharmaceutical composition is 5.0 to 7.0, and non-limiting examples include 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0 and any range between these point values. In some embodiments, the pH is 5.0 to 6.5 or 5.3 to 6.3.

In some embodiments, the pharmaceutical composition further comprises a liquid medium, e.g., water, physiological saline and glucose injection.

In some embodiments, the pharmaceutical composition comprises:
(a) 10 mg/mL to 180 mg/mL of the compound as shown in formula (I) or the pharmaceutically acceptable salt thereof, and (b) 10 mg/mL to 250 mg/mL of the arginine, wherein the pharmaceutical composition has a pH of 5.0 to 6.5.

In some embodiments, the pharmaceutical composition comprises:
(a) 30 mg/mL to 180 mg/mL of the compound as shown in formula (I) or the pharmaceutically acceptable salt thereof, and (b) 30 mg/mL to 200 mg/mL of the arginine, wherein the arginine is a combination of free arginine and arginine hydrochloride in a weight ratio of 1 : 0.1 to 1 : 20, and the pharmaceutical composition has a pH of 5.3 to 6.3.

In some embodiments, the pharmaceutical composition comprises:
(a) 10 mg/mL to 180 mg/mL of the compound as shown in formula (I) or the pharmaceutically acceptable salt thereof, (b) 10 mg/mL to 250 mg/mL of the arginine, and (c) 1 mg/mL to 80 mg/mL of sodium chloride, wherein the pharmaceutical composition has a pH of 5.0 to 6.5.

The present disclosure further provides a lyophilized formulation comprising the compound as shown in formula (I) or the pharmaceutically acceptable salt thereof, wherein the lyophilized formulation is obtained by freeze-drying the pharmaceutical composition comprising the compound as shown in formula (I) or the pharmaceutically acceptable salt thereof as described above.

The present disclosure further provides a reconstituted solution comprising the compound as shown in formula (I) or the pharmaceutically acceptable salt thereof, wherein the reconstituted solution is prepared by reconstituting the lyophilized formulation as described above.

The present disclosure further provides a method for preparing the reconstituted solution described above, wherein the method comprises the step of reconstituting the lyophilized formulation described above, wherein the solution used for the reconstitution is selected from, but not limited to water for injection, physiological saline or glucose solution.

The present disclosure further provides a lyophilized formulation comprising the compound as shown in formula (I) or the pharmaceutically acceptable salt thereof, wherein the formulation, upon reconstitution, forms the pharmaceutical composition as described above.

The present disclosure further provides a method for preparing a lyophilized formulation comprising the compound as shown in formula (I) or the pharmaceutically acceptable salt thereof, wherein the method comprises the step of freeze-drying the pharmaceutical composition as described above.

The present disclosure further provides an article of manufacture comprising a container, wherein the container contains the pharmaceutical composition, lyophilized formulation or reconstituted solution as described above. In some embodiments, the container is a neutral borosilicate glass tubular injection vial.

In some embodiments, the lyophilized formulation of the present disclosure is placed at 25°C, 60% RH for 1 month, wherein the reduction in purity of the compound as shown in formula (I) or the pharmaceutically acceptable salt thereof is less than 4.0%, which may be 4.0%, 3.9%, 3.8%, 3.7%, 3.6%, 3.5%, 3.4%, 3.3%, 3.2%, 3.1%, 3.0%, 2.9%, 2.8%, 2.7%, 2.6%, 2.5%, 2.4%, 2.3%, 2.2%, 2.1%, 2.0%, 1.9%, 1.8%, 1.7%, 1.6%, 1.5%, 1.4%, 1.3%, 1.2%, 1.1%, 1.0%, 0.9%, 0.8%, 0.7%, 0.6%, 0.5%, 0.4%, 0.3%, 0.2%, 0.1% or less.

In some embodiments, the lyophilized formulation of the present disclosure is placed at 25°C, 60% RH for 3 months, wherein the reduction in purity of the compound as shown in formula (I) or the pharmaceutically acceptable salt thereof is less than 5.0%, which may be 5.0%, 4.9%, 4.8%, 4.7%, 4.6%, 4.5%, 4.4%, 4.3%, 4.2%, 4.1%, 4.0%, 3.9%, 3.8%, 3.7%, 3.6%, 3.5%, 3.4%, 3.3%, 3.2%, 3.1%, 3.0%, 2.9%, 2.8%, 2.7%, 2.6%, 2.5%, 2.4%, 2.3%, 2.2%, 2.1%, 2.0%, 1.9%, 1.8%, 1.7%, 1.6%, 1.5%, 1.4%, 1.3%, 1.2%, 1.1%, 1.0%, 0.9%, 0.8%, 0.7%, 0.6%, 0.5%, 0.4%, 0.3%, 0.2%, 0.1% or less.

Provided is use of the pharmaceutical composition, lyophilized formulation and reconstituted solution of the present disclosure in the prevention and treatment of a disease caused by pathogenic bacteria in a mammal, including human. The disease is, for example, airway infectious diseases, urinary system infectious diseases, respiratory system infectious diseases, sepsis, nephritis, cholecystitis, oral infectious diseases, endocarditis, pneumonia, bone marrow membrane myelitis, otitis media, enteritis, empyema, trauma-related infectious diseases and opportunistic infections.

Provided is use of the pharmaceutical composition, lyophilized formulation and reconstituted solution of the present disclosure in the prevention and treatment of a disease caused by Gram-negative bacteria. The Gram-negative bacteria are preferably Gram-negative bacteria of intestinal bacteria *(E. coli, Klebsiella, Serratia, Enterobacter, Citrobacter, Morganella, Providencia, Proteus,* etc.), Gram-negative bacteria resident in the respiratory system *(Haemophilus, Moraxella,* etc.), and glucose-nonfermenting Gram-negative bacteria *(Pseudomonas aeruginosa, Pseudomonas* other than *P. aeruginosa, Stenotrophomonas, Burkholderia, Acinetobacter,* etc.).

As used herein, the term "about" or "approximately" means that the numerical value is within an acceptable error range for a particular value as determined by a person of ordinary skill in the art, which will depend in part on how the value is measured or determined (i.e., the limitations of the measurement system). For example, "about" may mean within 1 or more than 1 standard deviation, per the practice in the art. Alternatively, "about" or "substantially comprising" may mean a range of up to 20%. In addition, particularly with respect to biological systems or processes, the term may mean within at most an order of magnitude or within at most 5-fold of a value. Unless otherwise stated, when a particular value appears in the present application and claims, the meaning of "about" or "substantially comprising" shall be assumed to be within an acceptable error range for that particular value.

The numerical values in the present disclosure are measured values from instruments or calculated values derived from instrument measurements, which have a certain degree of error. In general, plus or minus 10% is within a reasonable error range. Of course, the context in which the numerical value is used needs to be considered. For example, with regard to the content of total impurities, the change in the error of the numerical value after measurement does not exceed plus or minus 10%, and may be plus or minus 9%, plus or minus 8%, plus or minus 7%, plus or minus 6%, plus or minus 5%, plus or minus 4%, plus or minus 3%, plus or minus 2% or plus or minus 1%, preferably plus or minus 5%.

"Weight-to-volume ratio" as used herein refers to the weight (in g) of the component contained per 100 mL of a liquid system, i.e., g/100 mL.

### Detailed Description of Embodiments

### Example 1: Solubility test of the compound as shown in formula (I)

Different solvent systems were prepared to test the solubility of the compound as shown in formula (I). The results are as shown in Table 1.

**Table 1 Solubility test**

| Solvent system | Charging amount (mg) | Dissolution Status | Solution pH after complete dissolution | Solubility |
|---|---|---|---|---|
| Purified water, 20 mL | 20.01 | Incomplete dissolution | / | / |
| Purified water, 250 mL | 20.01 | Complete dissolution | 3.6 | < 1 mg/ml |
| Hydrochloric acid (pH 1.0), 200 mL | 19.99 | Incomplete dissolution | / | / |
| Purified water + 1 M NaOH solution for pH adjustment, 4.9 ml | 500.4 | Complete dissolution | 5.08 | ≥ **100** mg/ml |

### Example 2

According to the formula composition in Table 2, the compound as shown in formula (I) was weighed, added to a small beaker, and suspended in water for injection. During stirring, the pH was adjusted to the target value using a buffer system solution or arginine. The protective excipients in Table 2 were added in proportion and dissolved with stirring, and then the concentration was adjusted to the drug concentration as shown in Table 2 by adding water for injection. Sterile filtration was performed, and an intermediate drug solution was obtained. A certain volume of the intermediate drug solution was aliquoted into vials, and the final sterile powder formulation was prepared by freeze-drying.

**Table 2 Composition of formulations**

| No. | Drug concentration | Protective excipient (weight-to-volume ratio) | Buffer system | Formulation pH |
|---|---|---|---|---|
| Formulation 1 | 100 mg/ml | 9% sucrose + 2.16% sodium chloride | Sodium hydroxide, hydrochloric acid | 5.85 |
| Formulation 2 | 100 mg/ml | 12% arginine | Sodium hydroxide, hydrochloric acid | 5.47 |
| Formulation 3 | 100 mg/ml | 9% sucrose + 2.16% sodium chloride | Sodium hydroxide, hydrochloric acid | 5.46 |
| Formulation 4 | 100 mg/ml | 9% sucrose + 5% mannitol | 10 mM histidine | 5.91 |
| Formulation 5 | 50 mg/ml | 12% arginine | 10 mM histidine | 5.80 |
| Formulation 6 | 100 mg/ml | 10% sorbitol | 10 mM histidine | 6.01 |
| Formulation 7 | 100 mg/ml | 7.8% arginine + 4.2% sucrose | Sodium hydroxide, hydrochloric acid | 5.76 |
| Formulation 8 | 100 mg/ml | 10% arginine + 1.4% sodium chloride | Sodium hydroxide, hydrochloric acid | 5.57 |

### Example 3

The powder formulation samples prepared in Example 2 were dried and then packaged under nitrogen flushing. The packaged samples were placed under accelerated conditions (25°C, 60% RH), and the contents of active substances and impurities in the samples were tested. The detection method was performed using a high-performance liquid chromatography system (chromatographic column: XBridge Shield PR18, 4.6 × 150 mm, 3.5 µm; mobile phase A: perchloric acid/water; mobile phase B: acetonitrile/methanol/perchloric acid; detection wavelength: 260 nm).

The detection results are as shown in the table below.

**Table 3 Stability test**

| No. | Condition | | Purity% | Δ Change% |
|---|---|---|---|---|
| Formulation 1 | Initial | | 97.93 | / |
| | 25°C, RH 60% | 14 days | 97.19 | -0.74 |
| | | 1M | 97.07 | -0.86 |
| | | 2 M | 96.49 | -1.44 |
| | | 3 M | 96.04 | -1.89 |
| Formulation 2 | Initial | | 98.13 | / |
| | 25°C, RH 60% | 14 days | 97.75 | -0.38 |
| | | 1M | 97.70 | -0.43 |
| | | 2 M | 97.31 | -0.82 |
| | | 3 M | 97.16 | -0.97 |
| Formulation 3 | Initial | | 97.84 | / |
| | 25°C, RH 60% | 14 days | 96.81 | -1.03 |
| | | 1M | 96.65 | -1.19 |
| | | 2 M | 96.30 | -1.54 |
| | | 3 M | 95.94 | -1.9 |
| Formulation 4 | Initial | | 95.72 | / |
| | 25°C, RH 60% | 7 days | 94.42 | -1.30 |
| | | 14 days | 93.28 | -2.44 |
| | | 1 M | 93.23 | -2.49 |
| Formulation 5 | Initial | | 95.58 | / |
| | 25°C, RH 60% | 7 days | 95.22 | -0.36 |
| | | 14 days | 94.75 | -0.83 |
| | | 1 M | 95.28 | -0.30 |
| | | 2 M | 94.76 | -0.82 |
| Formulation 6 | Initial | | 95.06 | / |
| | 25°C, RH 60% | 7 days | 84.82 | -10.24 |
| | | 14 days | 78.92 | -16.14 |
| Formulation 7 | Initial | | 94.86 | |
| | 25°C, RH 60% | 1M | 94.84 | -0.02 |
| | | 2 M | 94.32 | -0.54 |
| Formulation 8 | Initial | | 96.95 | |
| | 25°C, RH 60% | 1 M | 96.47 | -0.48 |
| | | 2 M | 96.59 | -0.36 |

After 2 months of placement under accelerated conditions, the formulations containing arginine showed the smallest change in purity (< 1%) and exhibited good stability. In contrast, the formulations with other protective excipients experienced a relatively significant decrease in purity.

### Example 4

According to the formula composition in Table 4, 500 mg of the compound as shown in formula (I) was weighed, added to a small beaker, and suspended in water for injection. During continuous stirring, 96 mg of arginine was added. After complete dissolution, the solution was adjusted to pH 5.4-5.8 with sodium hydroxide or hydrochloric acid. 609.49 mg of arginine hydrochloride was added. After complete dissolution, water for injection was added to make the total volume to 5 ml. Sterile filtration was performed, and an intermediate drug solution was obtained. The formulation solution was filled into vials, and the final sterile powder formulation was prepared by freeze-drying.

**Table 4 (Specification: 500 mg)**

| Component | Amount per vial (mg) |
|---|---|
| Compound as shown in formula (I) | 500 |
| Arginine | 96 |
| Arginine hydrochloride | 609.49 |
| Sodium hydroxide | Q.S. |
| Hydrochloric acid | Q.S. |
| Water for injection | Made up to 5 ml |
| pH of intermediate drug solution | 5.4-5.8 |

| | |
|---|---|
| Notes: [1] The total amount of arginine and arginine hydrochloride is calculated based on arginine, accounting for 12% of the formulation. [2] Water for injection is removed during the freeze-drying process. | |

The stability of the formulation under accelerated conditions (25°C, 60% RH) is as follows.

**Table 5 Stability test**

| Condition | | Purity% | Δ Change% |
|---|---|---|---|
| Initial | | 96.3 | / |
| 25°C, 60%RH | 1 M | 95.7 | -0.6 |
| | 2 M | 95.2 | -1.1 |
| | 3 M | 95.2 | -1.1 |

## Claims

1. A pharmaceutical composition, **characterized by** comprising a compound as shown in formula (I) or a pharmaceutically acceptable salt thereof, and arginine,

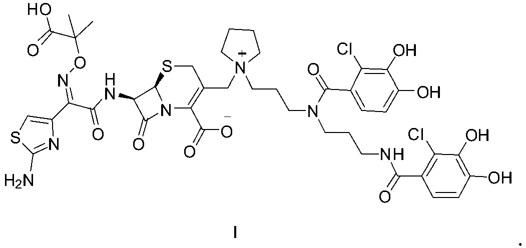

2. The pharmaceutical composition according to claim 1, **characterized in that** the compound as shown in formula (I) or the pharmaceutically acceptable salt thereof has a concentration of 1 mg/mL to 200 mg/mL, preferably 10 mg/mL to 180 mg/mL, more preferably 30 mg/mL to 180 mg/mL, and most preferably 30 mg/mL to 150 mg/mL.

3. The pharmaceutical composition according to claim 1 or 2, **characterized in that** the arginine has a weight-to-volume ratio of 0.01% to 30%, preferably 0.1% to 20%, and more preferably 1% to 15%.

4. The pharmaceutical composition according to any one of claims 1-3, **characterized in that** the arginine has a concentration of 1 mg/mL to 300 mg/mL, preferably 10 mg/mL to 250 mg/mL, more preferably 30 mg/mL to 200 mg/mL, and most preferably 30 mg/mL to 180 mg/mL.

5. The pharmaceutical composition according to any one of claims 1-4, **characterized in that** the compound as shown in formula (I) or the pharmaceutically acceptable salt thereof and the arginine are in a weight ratio of 1 : 0.1-1 : 20.

6. The pharmaceutical composition according to any one of claims 1-5, **characterized in that** the pharmaceutical composition further comprises other protective excipients, wherein the protective excipients are preferably selected from one or more of amino acids, saccharides, high-molecular-weight polysaccharides, cyclodextrins and inorganic salts, more preferably one or more of histidine, lysine, glycine, sucrose, glucose, lactose, trehalose, maltose, inositol, hydroxyethyl starch, PEG, dextran, sulfobutyl-β-cyclodextrin and sodium chloride.

7. The pharmaceutical composition according to any one of claims 1-6, **characterized in that** the pharmaceutical composition further comprises a buffer, wherein the buffer is preferably selected from an acetate buffer, a citrate buffer, a histidine buffer and a succinate buffer.

8. The pharmaceutical composition according to any one of claims 1-7, **characterized in that** the pharmaceutical composition further comprises a pH regulator, and preferably, the pH regulator is hydrochloric acid and/or sodium hydroxide.

9. The pharmaceutical composition according to any one of claims 1-8, **characterized in that** the pharmaceutical composition has a pH of 5.0 to 7.0, preferably 5.0 to 6.5, and more preferably 5.3 to 6.3.

10. The pharmaceutical composition according to any one of claims 1-9, **characterized in that** the pharmaceutical composition comprises:
(a) 10 mg/mL to 180 mg/mL of the compound as shown in formula (I) or the pharmaceutically acceptable salt thereof, and (b) 10 mg/mL to 250 mg/mL of the arginine, wherein the pharmaceutical composition has a pH of 5.0 to 6.5;
preferably, the pharmaceutical composition comprises:
(a) 30 mg/mL to 180 mg/mL of the compound as shown in formula (I) or the pharmaceutically acceptable salt thereof, and (b) 30 mg/mL to 200 mg/mL of the arginine, wherein the arginine is a combination of free arginine and arginine hydrochloride in a weight ratio of 1 : 0.1 to 1 : 20, and the pharmaceutical composition has a pH of 5.3 to 6.3; or
the pharmaceutical composition comprises:
(a) 10 mg/mL to 180 mg/mL of the compound as shown in formula (I) or the pharmaceutically acceptable salt thereof, (b) 10 mg/mL to 250 mg/mL of the arginine, and (c) 1 mg/mL to 80 mg/mL of sodium chloride, wherein the pharmaceutical composition has a pH of 5.0 to 6.5.

11. A lyophilized formulation, **characterized in that** the lyophilized formulation, upon reconstitution, forms the pharmaceutical composition according to any one of claims 1-10.

12. A lyophilized formulation, **characterized in that** the lyophilized formulation is obtained by freeze-drying the pharmaceutical composition according to any one of claims 1-10.

13. A reconstituted solution, **characterized in that** the reconstituted solution is prepared by reconstituting the lyophilized formulation according to claim 11 or 12.

14. Use of the pharmaceutical composition according to any one of claims 1-10, the lyophilized formulation of claim 11 or 12, or the reconstituted solution according to claim 13 in the preparation of a medicament for treating a disease caused by pathogenic bacteria in a mammal, **characterized in that** the disease is preferably airway infectious diseases, urinary system infectious diseases, respiratory system infectious diseases, sepsis, nephritis, cholecystitis, oral infectious diseases, endocarditis, pneumonia, bone marrow membrane myelitis, otitis media, enteritis, empyema, trauma-related infectious diseases and opportunistic infections.

15. Use of the pharmaceutical composition according to any one of claims 1-10, the lyophilized formulation according to claim 11 or 12, or the reconstituted solution according to claim 13 in the preparation of a medicament for treating a disease caused by Gram-negative bacteria.
